# EUROPEAN PATENT APPLICATION

(11) **EP 1 649 828 A1**
(43) Date of publication of application: **26.04.2006**
(21) Application number: 04733940.3
(22) Date of filing: 19.05.2004
(51) Int. Cl.: A61F 2/00, A61F 2/06, A61L 27/56

(54) **IN VIVO GRAFTING MATERIAL**

(30) Priority: 30.06.2003 JP 2003186475
(71) Applicant: Sumitomo Electric Industries, Ltd., Osaka-shi, Osaka 541-0041 (JP)
(72) Inventor: HAYASHI, Fumihiro; c/o Osaka Works of Sumitomo Ele, Osaka-shi, Osaka 5548511 (JP)
(74) Representative: Cross, Rupert Edward Blount
(86) International application number: PCT/JP2004/007120
(87) International publication number: WO 2005/000162

(57) **Abstract**

An intracorporeally implanting material comprising a composite body of a formed finely-porous or non-porous resin article, which is finely-porous, the pore diameter of which is smaller than 0.45 µm, or is non-porous and has vital tissue-blocking ability and a formed porous resin article, which is porous, the pore diameter of which is not smaller than 0.45 µm, and has vital tissue-penetrating ability. The material has a structure that a part of the formed porous resin article is exposed, and a vital tissue can be penetrated into the exposed part.

## Description

### TECHNICAL FIELD

The present invention relates to an intracorporeally implanting material used as a substitute for a vital tubular structure or septum lost by a disease or injury, such as an artificial blood vessel or a pericardial patch and having vital tissue-blocking ability. In particular, the present invention relates to an intracorporeally implanting material capable of inhibiting inflammation at a implanted site in addition to the vital tissue-blocking ability.

### BACKGROUND ART

A porous expanded polytetrafluoroethylene body (hereinafter abbreviated as "porous expanded PTFE body") obtained by stretching polytetrafluoroethylene (hereinafter abbreviated as "PTFE") has a microstructure comprising a great number of very fine fibrils and a great number of nodes connected to each other by the fibrils, and this microstructure forms a open-cell porous structure. The pore diameter and porosity of the porous expanded PTFE body can be optionally preset mainly by controlling conditions of stretching.

To the porous expanded PTFE body, are imparted properties and characteristics such as flexibility, fluid permeability, particle-collecting ability, filerability, low dielectric constant and low dielectric loss tangent by virtue of its porous structure, in addition to properties such as heat resistance and chemical resistance and surface properties such as low coefficient of friction, water repellency and tack-free property, which are possessed by the PTFE itself. The uses of the porous expanded PTFE body are widened to not only a general industrial field, but also a medical field or the like because of its particular properties and characteristics. Since the porous expanded PTFE body also has chemical stability, non-toxicity to a vital body, non-degradability and antithrombogenicity, it is an optimum material for uses directly touching an intracorporeal tissue.

Although the PTFE itself is a hard and brittle resin, the porous expanded PTFE body is permitted to flexibly change its form in conformity with the forms or the like of various vital tissues because the strength of fibrils thereof is strong, and it has good flexibility. Therefore, the porous expanded PTFE body is used as a porous body having a structure such as a flexible sheet or tube in a field of medical polymeric materials for patches, artificial blood vessels, catheters, etc.

Since the porous expanded PTFE body has a porous structure high in porosity, it is rich in flexibility, pliability and permeability and is suitable for use as an intracorporeally implanting material. PTFE is stretched at a draw ratio of from 2 or 3 times to several tens times, whereby flexibility is imparted to the porous expanded PTFE body, and a porous structure of a high porosity is formed, so that a surrounding vital tissue can enter into or permeate through the porous structure in a vital body, thereby creating a good cured state in one with the vital tissue.

On the other hand, in an intracorporeally implanting material required to prevent the invasion and adhesion of a surrounding tissue and a cured tissue, it is necessary to impart vital tissue-blocking ability. For example, an intracorporeally implanting material used for repairing a pericardium, pleura, diaphragm, peritoneum, tendovagina or the like, such as a patch material, is required to have the vital tissue-blocking ability. The reason for it is that the pericardium, pleura, diaphragm, peritoneum or the like fulfills a role of screening an organ such as the heart, lung or digestive organ from its surrounding tissue to cause such organ to retain free function in the coelom.

For example, a pericardial patch having the vital tissue-blocking ability is used in a bypass surgery of a coronary artery that is conducted by cutting the thorax to expose the heart, or the like. The pericardial patch fulfills a role as the diaphragm preventing a surrounding tissue or cured tissue from entering into the heart to adhere thereto after the surgery. The use of the pericardial patch having the vital tissue-blocking ability has an advantage that access to the heart is made very easy in a frequent bypass surgery.

A non-porous formed resin article or a finely-porous formed resin article having a fine pore diameter to such an extent that invasion of a connective tissue is difficult to occur is used as such an intracorporeally implanting material having the vital tissue-blocking ability. In uses such as the pericardial patch, the form of the resin molding is generally in the form of a sheet (film). As a resin making up the formed resin article, PTFE is extremely useful for the prevention of adhesion because it is excellent in tack-free property. In particular, a finely-porous expanded PTFE body controlled to a fine pore diameter that invasion of a tissue is hard to occur is also good in surgery-operating property because it is flexible.

On the other hand, the porous expanded PTFE body generally involves such problems that orientation of fibrils occurs in an extrusion step and a stretching step to cause anisotropy in strength and that it is easy to tear or break upon suture with a suture. In particular, a finely-porous expanded PTFE body having a fine pore diameter and produced at a low draw ratio is low in the strength of fibrils and thus has a strong tendency to become insufficient in tear strength.

In order to overcome such problems as described above, there have heretofore been proposed a method that at least 2 porous expanded PTFE sheets are integrally laminated with their stretching directions crossed each other at an optional angle (for example, Japanese Patent Application Laid-Open No. 54-90897), a method that a porous expanded PTFE body is subjected to a heat treatment to form node-aggregating parts and node-non-aggregating parts, and the node-aggregating parts are integrally connected to form a continuous pattern surrounding the node-non-aggregating parts (for example, Japanese Patent Application Laid-Open No. 7-82399). In these methods, production conditions are controlled so as to make a pore diameter small, whereby a laminate or porous expanded PTFE body which has vital tissue-blocking ability and is suitable for use as an intracorporeally implanting material can be obtained.

In the case where the porous expanded PTFE body is used as the intracorporeally implanting material, there has been proposed a method that the pore diameter of the porous expanded PTFE body is controlled within a range of 0.05 to 0.5 µm to achieve the vital tissue-blocking ability while retaining humoral communicating ability, and such a finely-porous expanded PTFE sheet and a porous expanded PTFE sheet having a pore diameter of, for example, 1 µm are laminated, thereby improving balance among vital tissue-blocking ability, flexibility and strength in the whole intracorporeally implanting material. (Japanese Patent Application Laid-Open No. 9-173438).

### DISCLOSURE OF THE INVENTION

Pericardial patches made of a porous expanded PTFE body, which are currently commercially sold, are designed to be non-toxic and flexible and also have good vital tissue-blocking ability in clinic. However, it is indicated that the pericardial patches made of the porous expanded PTFE body are easy to cause inflammation at an implanted site and its surrounding site (for example, Yamanashi University, Shoji Suzuki, et al., "An Example of Postoperative Intractable Pericardial Fluid Retention Considered by EPTFE Artificial Pericardium", 99-th Kanto-Koshinetsu Local Meeting in Japan Thoracic Surgery Society, 1996, Tokyo).

In fact, the present inventors carried out a subcutaneously implanting test against rats using a patch (implant) made of a finely-porous expanded PTFE body. As a result, it was confirmed that a cicatricial tissue around an implanted site becomes thick, and a humor is retained around the implant to cause inflammation. Such an inflammatory tendency becomes greater as the pore diameter of the porous expanded PTFE body is smaller. In particular, a formed non-porous resin article such as an unexpanded PTFE sheet is low in flexibility and pliability and thus has no humoral communicating ability, so that inflaming ability becomes higher.

According to the conventional finding in this technical field, it was considered that the inflaming ability of an implanting material having the vital tissue-blocking ability is extremely difficult or impossible to reduce the inflaming ability while substantially retaining the vital tissue-blocking ability in the cause of its non-porous nature or finely-porous nature. Even when a finely-porous expanded PTFE sheet having a small pore diameter and a porous expanded PTFE sheet having great pore diameter are laminated, it is difficult to inhibit inflammation by the hypertrophy of a cicatricial tissue and the retention of a humor when the finely-porous expanded PTFE sheet having the vital tissue-blocking ability is present on the whole surface of the laminate (multi-layer sheet).

It is an object of the present invention to provide a novel intracorporeally implanting material, which is composed of a formed article of a synthetic resin, substantially has vital tissue-blocking ability and moreover can markedly inhibit inflammation caused by the hypertrophy of the cicatricial tissue and the retention of the humor at an implanted site and its surrounding site.

The present inventors have carried out an extensive investigation with a view toward achieving the above object. As a result, it has been found that a formed finely-porous or non-porous resin article having vital tissue-blocking ability and a formed porous resin article having vital tissue-penetrating ability are composited, and at this time, a part of the formed porous resin article is exposed so as to permit penetration of a vital tissue into the exposed part, whereby inflammation caused by the hypertrophy of a cicatricial tissue and the retention of a humor at an implanted site and its surrounding site can markedly inhibited while substantially retaining the vital tissue-blocking ability.

The intracorporeally implanting material according to the present invention substantially has the vital tissue-blocking ability as a whole. However, the formed porous resin article having a pore size that a vital tissue can be penetrated is exposed from a part thereof, whereby the implanted site and its surrounding cured tissue can freely penetrates into the exposed part to be integrated and fixed, thereby greatly reducing the inflammation caused by the hypertrophy of the cicatricial tissue and the retention of the humor. In addition, since the intracorporeally implanting material according to the present invention is a composited material, the properties of the respected formed resin articles composited can be controlled, whereby balance between flexibility and strength can also be controlled. The present invention has been led to completion on the basis of these findings.

According to the present invention, there is provided an intracorporeally implanting material comprising a composite body of a formed finely-porous or non-porous resin article (A), which is finely-porous, the pore diameter of which is smaller than 0.45 µm, or is non-porous and has vital tissue-blocking ability and a formed porous resin article (B), which is porous, the pore diameter of which is not smaller than 0.45 µm, and has vital tissue-penetrating ability, wherein the material has a structure that a part of the formed porous resin article (B) is exposed, and a vital tissue can be penetrated into the exposed part.

### BRIEF DESCRIPTION OF THE DRAWINGS

Fig. 1 is a front view illustrating an exemplary intracorporeally implanting material according to the present invention.
Fig. 2 is a cross-sectional view illustrating the exemplary intracorporeally implanting material according to the present invention.
Fig. 3 schematically illustrates another exemplary intracorporeally implanting material according to the present invention, in which Fig. 3(a) illustrates a member A cut out into a frame form, Fig. 3(b) illustrates a member B cut out into a rectangular form, and Fig. 3(c) illustrates a laminated structure.

### BEST MODE FOR CARRYING OUT THE INVENTION

Materials of the formed finely-porous or non-porous resin article (A) and formed porous resin article (B) used in the present invention may be suitably selected from a fluorocarbon resin, a polyester resin, polypropylene, polyethylene, a polyamide resin and the like.

Among these resin materials, fluorocarbon resins such as polytetrafluoroethylene (PTFE), tetrafluoroethylene-hexafluoropropylene copolymers (FEPs), tetrafluoroethylene-perfluoroalkyl vinyl ether copolymers (PFAs), polyvinylidene fluoride resins and tetrafluoroethylene-ethylene copolymers (ETFEs) are preferred. Among the fluorocarbon resins, PTFE is preferred because they have properties such as chemical stability, non-toxicity to a vital body, non-degradability and antithrombogenicity. PTFE is preferably used in the form of a formed non-porous resin article or porous expanded PTFE body.

The formed non-porous resin article can be produced by ordinary extrusion or the like. In order to produce the formed non-porous PTFE article, a mixture of unsintered PTFE powder (fine powder for paste extrusion) and a lubricant (for example, solvent naphtha, petroleum or the like) is compressed in a cylinder to perform it into a columnar form, and the resultant perform (billet) is then poured into an extrusion cylinder and pressurized by a ram to extrude it through a die, thereby producing an extruded article in the form of a sheet or rod. In order to obtain the sheet-like extruded article, a T-die is connected to the tip of the extrusion cylinder, and a die opened in a circular form is used to obtain the rod-like extruded article.

The sheet-like or rod-like extruded article obtained in the extrusion step is rolled by means of a rolling mill such as a roll or press before the lubricant is vaporized out to produce a rolled sheet having a predetermined thickness. After the lubricant is removed, the rolled sheet is heated to a temperature not lower than a melting point (327°C) of PTFE in a fixed state, whereby a sheet-like formed non-porous PTFE article can be obtained.

The formed finely-porous or porous resin article is preferably produced by a stretching process. A porous expanded PTFE body is most preferred from the viewpoint of biological safety. Therefore, a production process of the formed porous resin article by the stretching process is described taking the porous expanded PTFE body as an example.

The porous expanded PTFE body can be produced in accordance with, for example, the process described in Japanese Patent Publication No. 42-13560. Fine PTFE powder is first mixed with a lubricant, and the resultant mixture is extruded into the form of a sheet or tube. After the extruded product is worked by rolling or the like as needed, it is stretched in at least one direction. After the stretching, the porous expanded PTFE body is heated to a temperature of at least 327°C that is a sintering temperature while fixing it so as not to cause shrinkage, thereby sintering and fixing the stretched structure. The porosity, pore diameter and fiber length (average fibril length) of the porous expanded PTFE body can be optionally preset by controlling a draw ratio and a stretch strain rate. As described above, PTFE is preferred as a resin material forming the formed resin articles according to the present invention even in that the porous structure can be freely preset.

The formed finely-porous or non-porous resin article (A) used in the present invention is a formed resin article that is non-porous or has a pore diameter smaller than 0.45 µm in the case of a finely-porous article and vital tissue-blocking ability. In the case of the formed finely-porous resin article, the penetration of cells can be prevented so far as the pore diameter thereof is not higher than 0.50 µm. However, thick fasciculi may be penetrated in some cases. Therefore, the formed finely-porous resin article having a pore diameter smaller than 0.45 µm is used in the present invention for the purpose of achieving sufficient vital tissue-blocking ability. The formed finely-porous resin article having a pore diameter smaller than 0.45 µm can block penetration of particles having a particle diameter not smaller than 0.45 µm. A formed finely-porous resin article having a pore diameter of 0.05 to 0.45 µm is preferably used in uses of which humoral communicating ability is required.

On the other hand, the formed non-porous resin article is most excellent in the respect of the vital tissue-blocking ability. The formed finely-porous resin article having a pore diameter smaller than 0.45 µm is also excellent in the respect of the vital tissue-blocking ability. Since the intracorporeally implanting material according to the present invention comprises a composite body of a formed finely-porous or non-porous resin article (A) and a formed porous resin article (B), and a part of the formed porous resin article (B) is exposed in such a manner that a vital tissue can be penetrated into the exposed part, a formed non-porous resin article or a formed finely-porous resin article having a pore diameter smaller than 0.05 µm may be preferably used as a formed resin article having vital tissue-blocking ability.

When the formed finely-porous resin article is a finely-porous expanded PTFE body, its bubble point is preferably higher than 0.70 kgf/cm², more preferably at least 1.50 kgf/m² as measured with isopropyl alcohol from the viewpoint of the vital tissue-blocking ability.

On the other hand, since the formed porous resin article (B) has a role that a cured tissue freely penetrates into or permeates through it, thereby imparting a function that a vital tissue is integrated with and fixed to the intracorporeally implanting material, it is necessary that the formed resin article is a porous material having a pore diameter not smaller than 0.45 µm, into which particles having a particle diameter not smaller than 0.45 µm can penetrate. When the formed porous resin article (B) is a porous expanded PTFE body, its bubble point is preferably not higher than 0.70 kgf/cm², more preferably not higher than 0.50 kgf/m², particularly preferably not higher than 0.30 kgf/m² as measured with isopropyl alcohol from the viewpoint of the vital tissue-penetrating ability.

The formed porous resin article (B) used in the present invention preferably has a pore diameter not smaller than 1 µm in such a manner that particles having a particle diameter not smaller than 1 µm can penetrate. When the formed porous resin article (B) is a porous expanded PTFE body having a pore diameter not smaller than 1 µm, its bubble point is not higher than 0.50 kgf/cm². When the pore diameter of the formed porous resin article (B) is not smaller than 1 µm, it can be confirmed by observation through an electron microscope that a cell tissue penetrates into the porous structure, and the tissue becomes continuous to an extent that a boundary line with its surrounding tissue is indistinguishable. As described above, the formed porous resin article (B) having a pore diameter not smaller than 1 µm is markedly high in anchoring effect compared with a formed porous resin article having a pore diameter smaller than it.

The formed porous resin article (B) used in the present invention more preferably has a pore diameter not smaller than 3 µm in such a manner that particles having a particle diameter not smaller than 3 µm can penetrate. When the formed porous resin article (B) is a porous expanded PTFE body having a pore diameter not smaller than 3 µm, its bubble point is not higher than 0.30 kgf/cm². When the pore diameter of the formed porous resin article (B) is not smaller than 3 µm, whereby the particles having a particle diameter not smaller than 3 µm can penetrate, the penetrability of cells into the porous structure is more improved, and a capillary within the porous structure is also in a very developed state. On the other hand, a surrounding tissue covering the implanting material becomes very thin to create a stat that the inflaming ability is lowest.

When the pore diameter of the formed porous resin article (B) is not smaller than 5 µm or 10 µm, whereby the particles having a particle diameter not smaller than 5 µm or 10 µm can penetrate, the anchoring effect becomes extremely high. When the formed porous resin article (B) is a porous expanded PTFE body having a pore diameter not smaller than 10 µm, its bubble point is not higher than 0.15 kgf/cm².

The pore diameter of the formed porous resin article (B) can be controlled to preferably 1 to 15 µm, more preferably 3 to 15 µm, or not smaller than 5 µm or 10 µm as needed. The porosity of the formed porous resin article (B) is preferably not lower than 50%, more preferably not lower than 70%, particularly preferably not lower than 80%.

The forms of the intracorporeally implanting material and the respective formed resin articles may be suitably determined according to an implanting site or the like. However, it is generally in the form of a sheet or tube. When the intracorporeally implanting material is used as a patch material such as a pericardial patch material, it is preferably in the form of a sheet. The form and dimensions of the intracorporeally implanting material according to the present invention may be variously designed according to an implanting site and its uses. When it is in the form of a sheet, the thickness thereof is generally not greater than 3 mm, preferably not greater than 1 mm, more preferably not greater than 0.5 mm in consideration of surgery-operating property. The thickness of each of the formed finely-porous or non-porous resin article (A) and formed porous resin article (B) in the form of a sheet is preferably not greater than 1.5 mm, more preferably not greater than 500 µm, still more preferably not greater than 250 µm. The lower limit thereof is preferably 30 µm, more preferably 40 µm, particularly preferably 50 µm.

The intracorporeally implanting material according to the present invention comprises a composite body of a formed resin article (A), which is finely-porous, the pore diameter of which is smaller than 0.45 µm, or is non-porous and has vital tissue-blocking ability, and a formed resin article (B), which is porous, the pore diameter of which is not smaller than 0.45 µm, and has vital tissue-penetrating ability. Such a composite body can be generally produced by separately making the formed finely-porous or non-porous resin article (A) and the formed porous resin article (B) and then laminating them on each other to bond or fusion-bond them. Alternatively, a process, in which the formed finely-porous or non-porous resin article (A) is made in advance, and this resin article (A) is then partially stretched, thereby partially forming a formed resin portion, which is porous, the pore diameter of which is not smaller than 0.45 µm, and has vital tissue-penetrating ability, may also be adopted.

The intracorporeally implanting material according to the present invention is composed of the above-described composite body and has a structure that a part of the formed porous resin article (B) is exposed, and a vital tissue can be penetrated into the exposed part. A specific example of the intracorporeally implanting material will be described taking sheet-like formed resin articles as an example.

The intracorporeally implanting material according to the present invention comprises a composite body of a formed finely-porous or non-porous resin article (A), which is finely-porous, the pore diameter of which is smaller than 0.45 µm, or is non-porous and has vital tissue-blocking ability and a formed porous resin article (B), which is porous, the pore diameter of which is not smaller than 0.45 µm, and has vital tissue-penetrating ability, and has a structure that a part of the formed porous resin article (B) is exposed, and a vital tissue can be penetrated into the exposed part.

An example of the composite body having the structure that a part of the formed porous resin article (B) is exposed, and a vital tissue can be penetrated into the exposed part includes a composite body (multi-layer sheet) obtained by laminating a formed finely-porous or non-porous resin article (A) and a formed porous resin article (B), which are both in the form of a sheet, and having a structure that a part of the sheet-like formed porous resin article (B) extends outside a peripheral edge portion of the sheet-like formed finely-porous or non-porous resin article (A) in an exposed state.

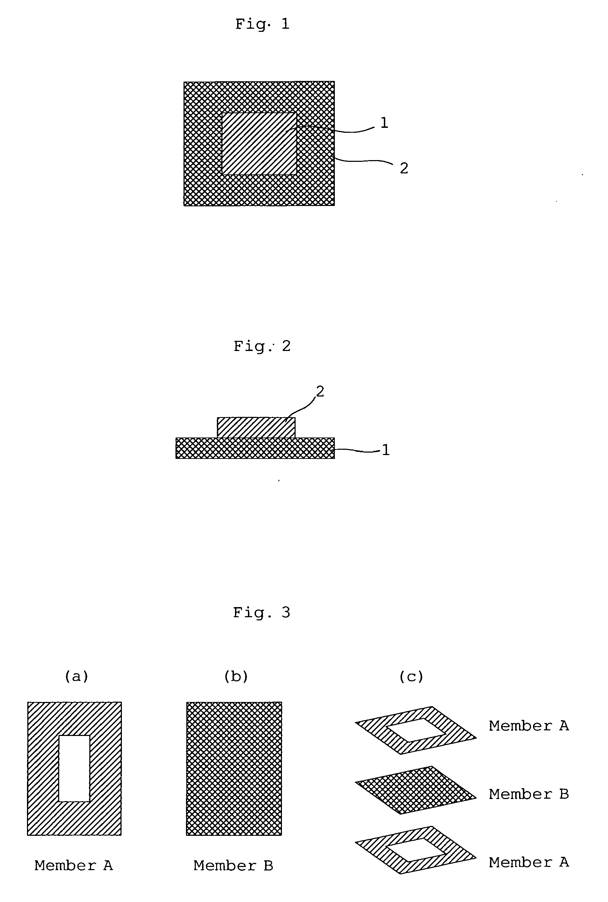

Fig. 1 is a front view of an example of the composite body obtained by laminating the sheet-like formed finely-porous or non-porous resin article (A) and the sheet-like formed porous resin article (B), and Fig. 2 is a cross-sectional view thereof. When a sheet-like formed finely-porous or non-porous resin article (A) 1 having a relatively small area and a sheet-like formed porous resin article (B) 2 having a relatively large area are superimposed and laminated on each other, a part of the formed porous resin article (B) 2 is exposed by itself around the laminated part. A cured tissue and its surrounding vital tissue penetrate into the porous structure of this exposed part. As a result, the intracorporeally implanting material is integrated with the vital tissue. This exposed part is also excellent in humoral communicating ability. Therefore, the intracorporeally implanting material of such a structure inhibits the hypertrophy of a cicatricial tissue and the retention of a humor at an implanted site and a periphery thereof and is thus low in inflammatory tendency.

In the composite body as illustrated in Figs. 1 and 2, the size and form of the formed finely-porous or non-porous resin article (A) 1 having the vital tissue-blocking ability can be designed in conformity with the form or the like of an implanted site. The tear strength of the formed porous resin article (B) 2 exposed around the laminated part can be enhanced by raising the draw ratio thereof. Such a porous resin article is also excellent in flexibility.

The above-described composite body (multi-layer sheet) has the structure that a part of the sheet-like formed porous resin article (B) 2 extends outside a peripheral edge portion of the sheet-like formed finely-porous or non-porous resin article (A) 1 in an exposed state. When the sheet-like formed finely-porous or non-porous resin article (A) 1 is rectangular as shown in Figs. 1 and 2, a part of the sheet-like formed porous resin article (B) 2 may extend outside at least a part of the peripheral edge part thereof. Even when the sheet-like formed finely-porous or non-porous resin article (A) 1 is in another form such as a circle, a part of the sheet-like formed porous resin article (B) 2 may also extend outside at least a part of the peripheral edge part thereof. An area of the part (exposed part) where the sheet-like formed porous resin article (B) 2 is present outside the peripheral edge part of the sheet-like formed finely-porous or non-porous resin article (A) 1 is generally at least 5%, preferably at least 10% of the whole area of the sheet-like formed porous resin article (B) 2, and the upper limit thereof is generally 70%, preferably 60%, more preferably 50%.

From the viewpoint of improving the balance between tissue-blocking ability and curing ability, the intracorporeally implanting material preferably has a structure that a part of the sheet-like formed porous resin article (B) extends outside the whole peripheral edge part of the sheet-like formed finely-porous or non-porous resin article (A). A width of the part (exposed part) where a part of the sheet-like formed porous resin article (B) 2 is present outside the peripheral edge part of the sheet-like formed finely-porous or non-porous resin article (A) 1 may be suitably determined in consideration of the whole size of the intracorporeally implanting material, the vital tissue-blocking ability, the condition of a vital tissue at an implanted site, etc. However, it is generally at least 3mm, preferably at least 5 mm, more preferably at least 10 mm. The upper limit of this width is generally 30 mm, preferably 25 mm, more preferably 20 mm. However, since the width of the part where a part of the sheet-like formed porous resin article (B) 2 is present outside the peripheral edge part of the sheet-like formed finely-porous or non-porous resin article (A) may be selected according to an application site of the intracorporeally implanting material, or the like, this width is not limited to the above range. Two sheet-like formed finely-porous or non-porous resin articles (A) having an area smaller than the sheet-like formed porous resin article (B) may also be arranged in a sandwiched state on both surfaces of the formed porous resin article (B).

As a specific example of another composite body having the structure that a part of the formed porous resin article (B) is exposed, and a vital tissue can be penetrated into the exposed part, may be mentioned a composite body obtained by laminating a formed finely-porous or non-porous resin article (A) and a formed porous resin article (B), which are both in the form of a sheet, and having a structure that a part of the sheet-like formed porous resin article (B) is exposed through an opening formed in the sheet-like formed finely-porous or non-porous resin article (A).

As the composite body of the structure that the opening is provided as described above, is preferred a composite body having a structure that a sheet-like formed porous resin article (B) is held between 2 sheet-like formed finely-porous or non-porous resin articles (A), and a part of the sheet-like formed porous resin article (B) is exposed through openings respectively formed in the two sheet-like formed finely-porous or non-porous resin articles (A).

For example, when openings are respectively formed in 2 sheet-like formed finely-porous or non-porous resin articles (members A), and a sheet-like formed porous resin article (member B) is held between these two sheets to laminate them as illustrated in Fig. 3, an intracorporeally implanting material of a 3-layer structure, in which the formed porous resin article (B) is exposed by itself through the openings, can be obtained.

The size and form of the opening can be suitably determined in consideration of the form of an implanted site and the balance with the vital tissue-blocking ability. Such an opening may be formed at not only one position, but also plural positions. An area of the opening is generally at least 20 mm², preferably at least 100 mm², more preferably at least 200 mm², particularly preferably at least 2,000 mm². The upper limit of the area of the opening is preferably 4,000 mm², more preferably 3,000 mm², particularly preferably 2,500 mm². The area of the opening is preferably at least 5%, more preferably at least 10% of the whole area, and the upper limit thereof is generally 70%, preferably 60%, particularly preferably 50%. However, since the area of the opening can be determined according to the size and form of the intracorporeally implanting material, an application site, etc., this area is not limited to the above range.

The laminate of the 3-layer structure as illustrated in Fig. 3 are high in tear strength and thus inhibits the tendency to be torn by suture because the formed porous resin article (B) is composited. Since the formed porous resin article (B) is exposed by itself, a vital tissue can be penetrated into this portion to be integrated. Since this exposed part is present, integration with the vital tissue is advanced to inhibit the hypertrophy of a cicatricial tissue and also the retention of a humor. As a result, the intracorporeally implanting material can be provided as a material that inflammatory tendency is markedly reduced. A part of the formed porous resin article (B) may also be caused to extend outside a peripheral edge part of the sheet-like formed finely-porous or non-porous resin article (A) in addition to the provision of the opening.

In order to laminate and integrate 2 or more sheet-like formed resin articles, a method such as pressing at a high temperature or lamination with an adhesive resin may be adopted. For example, when PTFE is used as a resin material, a finely-porous or non-porous PTFE sheet and a porous expanded PTFE sheet can be bonded by pressing them at a temperature not lower than the melting point of PTFE. They may also be bonded by interposing a hot-melt fluorocarbon resin such as FEP or PFA between such layers to melt and penetrate the hot-melt fluorocarbon resin at an interlaminar interface.

The intracorporeally implanting material according to the present invention can be integrated with a vital tissue (cured tissue) penetrated into a part of the formed porous resin article, which has been partially exposed, while substantially retaining the vital tissue-blocking ability, thereby inhibit the hypertrophy of a cicatricial tissue and the retention of a humor at an implanted site and its surrounding site.

In the intracorporeally implanting material according to the present invention, its tear strength can also be enhanced by compositing the respective formed resin articles by lamination, thereby making a suture operation easy. The formed porous resin article partially exposed can be integrated with the vital tissue, thereby firmly holding the intracorporeally implanting material within the vital body. The portion of the formed finely-porous or non-porous resin article having the vital tissue-blocking ability can prevent adhesion to an implanted site and excess penetration of the vital tissue into the implanted site by bringing it into contact with the implanted site.

In the intracorporeally implanting material according to the present invention, at least one of the portion of the formed finely-porous or non-porous resin article (A) having the vital tissue-blocking ability and the portion of the formed porous resin article (B) that a cured tissue freely penetrates into and permeates through the portion to be integrated with and fixed to the vital tissue can be colored in such a manner that such portions can be clearly distinguished from each other. Examples of a coloring method include a method of containing a pigment such as an inorganic salt of cobalt blue in a formed resin article; a method of applying such a pigment to a surface of the formed resin article; and a method of combining these methods.

More specifically, when the formed finely-porous resin article (A) or formed porous resin article (B) is a porous expanded PTFE body, examples of the coloring method include a method, in which a compound obtained by dispersing the pigment in PTFE powder that is a raw material is used to form a porous expanded PTFE body; and a method, in which a coating liquid with the pigment dispersed therein is applied to a surface of an unsintered porous expanded PTFE body and dried, and the porous PTFE body is then heated to a temperature not lower than the melting point of PTFE to sinter it.

Any one of the formed finely-porous or non-porous resin article (A) and the formed porous resin article (B) may be colored, or both may be colored with the kinds or densities of colors varied. The color tone of the formed finely-porous or non-porous resin article (A) is caused to differ from the color tone of the formed porous resin article (B) by coloring, whereby the functions of the respective parts making up the intracorporeally implanting material according to the present invention can be clearly distinguished, and in turn, the operation of an implanting surgery can be made easy.

### EXAMPLES

The present invention will hereinafter be described more specifically by the following Examples and Comparative Examples. Since a method of verifying the principle of the present invention by model experiments using small animals is adopted in the following Examples, the intracorporeally implanting materials were produced in sizes and forms convenient for evaluation. The present invention is not limited to these examples only. Physical properties were measured in accordance with the following respective methods.

### (1) Pore diameter:

The pore diameter means a pore diameter represented by a minimum particle diameter of particles that are captured without very penetrating into a material wall when a sample is used as a separation membrane. More specifically, after suspension containing spherical particles of respective average particle diameters were used to conduct filtration through a finely-porous or porous sheet, a minimum particle diameter that at least 90% of the particles can be captured was regarded as a pore diameter.

### (2) Bubble point:

After a porous expanded PTFE body was immersed in isopropyl alcohol to fill pores in a tube wall with isopropyl alcohol, a pressure was measured at the time bubbles came out of the other surface for the first time when an air pressure was gradually applied from one surface.

### (3) Porosity:

A volume was determined from a difference between a dry weight of a porous expanded PTFE body and its weight in water. A volume of the resin was calculated out from the dry weight and a true specific gravity of PTFE regarded as 2.25 g/cc. A proportion of a void volume obtained by subtracting the volume of the resin, that is occupied in the whole volume, was indicated by % to regard it as a porosity.

### (4) Evaluation method of implanting:

SD rats (male) aged 11 weeks were used as experimental animals. Two samples were subcutaneously implanted in the back of an animal. In order to reduce the influence of an individual difference, the samples of the same kind were implanted in the animal so as not to overlap each other. At this time, the respective samples were implanted at 2 positions of the vicinity of the broadest muscle of the back and the vicinity of the gluteus maximus muscle so as to give n = 3 (total n = 6). The samples implanted were taken out after 4 weeks elapsed. In Comparative Example 4, only one sample of Comparative Example 4 was implanted in 4 rats to make evaluation.

### (5) Evaluation as to inflammatory tendency:

A sample was subcutaneously implanted in the back of a rat, and the sample was taken out together with its surrounding tissue after 4 weeks elapsed after the wound was closed to visually observe the condition of the tissue, and the like. After a part of the sample was cut out to immobilize it with formalin, it was replaced by anhydrous paraffin to be embedded in a paraffin block. After a segment having a thickness of 7 µm was placed on a preparation to conduct HE staining, the segment was observed through an electron microscope. As the evaluation as to inflammatory tendency, it was judged by whether the surrounding tissue was thick or thin. Since there were cases where a mucoidal humor was collected between the implant and the surrounding tissue in some samples by strong inflammation, such cases were counted.

### (6) Evaluation method of the degree of integration with a vital tissue as to a portion to which a function of being integrated with and fixed to the vital tissue was imparted:

A sample taken out of the experimental animal was fixed to a flat plate with an adhesive in a state that the surrounding tissue adhered thereto. The portion into which the cured tissue had freely penetrated, and to which a function of being integrated with and fixed to the vital tissue had been imparted, was adjusted together with the surrounding tissue adhered thereof so as to give a width of 10 mm. Thereafter, the porous expanded PTFE body was partially separated from the tissue with a pair of tweezers to form a tong hold, thereby pulling the sample by 25 mm at a rate of 20 mm/min by a 180°-peel method to peel it.

An average value of values obtained by dividing a maximum load recorded at this time by the width (10 mm) of the sample was regarded as peel strength. As a tensile tester was used an Autograph AG500E manufactured by Shimadzu Corporation, and the test was conducted while dropping physiological saline so as not to dry the sample in a series of measuring operations. In cases where a mucoidal humor was collected between the sample and the surrounding tissue by strong inflammation, the peel strength was not measured because the surrounding tissue dissociated from the sample. The higher the peel strength, the higher the tendency to be integrated.

By the observation of the pathological segments used in the evaluation as to inflammatory tendency, the degree of integration was judged by means of the degree of penetration of the tissue. Whether penetration of collagen fasciculi or the like that were impossible to be observed through an electron microscope occurred or not was judged by conducting critical point drying and then observing a section of the porous body through a scanning electron microscope.

### Example 1

After about 20 parts by weight of naphtha was blended into 100 parts by weight of fine PTFE powder (product of Asashi Glass Co., Ltd., trade name "CD4"), and the resultant blend was left to stand for at least 5 hours to be aged, the blend was extruded into a sheet through a T-die and further rolled by means of a rolling mill. The rolled product was heated to a temperature not lower than 340°C in a state that the shape of the formed article was fixed, thereby sintering it to make a non-porous and translucent PTFE sheet having a thickness of about 40 µm. The non-porous PTFE sheet was cut out into a frame form that is in the form of a rectangle 40 mm long and 30 mm wide and has an opening 20 mm long and 10 mm wide at its central portion as illustrated in Fig. 3(a), thereby providing members A (vital tissue-blocking members).

On the other hand, a porous expanded PTFE sheet (product of Sumitomo Electric Industries, Ltd., trade name "Poreflon WP-1000", thickness: about 100 µm, particle diameter capable of being captured: at least 10 µm) having a pore diameter of 10 µm was cut out into a rectangular form 40 mm long and 30 mm wide as illustrated in Fig. 3(b), thereby providing a member B (vital tissue-integrating member).

As illustrated in Fig. 3(c), one sheet of member B was held between 2 sheets of member A, and they were held between stainless steel-made plates heated to at least 360°C, thereby heating and fusion-bonding them while pressing them to be integrated. An intracorporeally implanting material having a portion where the porous expanded PTFE sheet was exposed by itself was produced in such a manner.

With respect to implanted samples implanted in rats, the penetration of a vital tissue into the member B and the degree of integration with a surrounding tissue were observed. As a result, the penetration of cells was good, and a capillary was also developed. In SEM observation, the vital tissue became completely continuous within the surrounding tissue and the porous wall, and no boundary line between a tissue within the wall and an outer tissue could be distinguished. The results are shown in Table 1.

### Example 2

An intracorporeally implanting material was produced in the same manner as in Example 1 except that a porous expanded PTFE sheet (product of Sumitomo Electric Industries, Ltd., trade name "Poreflon WP-300", thickness: about 100 µm, particle diameter capable of being captured: at least 3 µm) having a pore diameter of 3 µm was used as the member B.

With respect to implanted samples implanted in rats, the penetration of a vital tissue into the member B and the degree of integration with a surrounding tissue were observed. As a result, the penetration of cells was good, and a capillary was also developed. In SEM observation, the vital tissue became completely continuous within the surrounding tissue and the porous wall, and no boundary line between a tissue within the wall and an outer tissue could be distinguished. The results are shown in Table 1.

### Example 3

An intracorporeally implanting material was produced in the same manner as in Example 1 except that a commercially available porous PTFE sheet (product of Sumitomo Electric Industries, Ltd., trade name "Poreflon WP-100", thickness: about 100 µm, particle diameter capable of being captured: at least 1 µm) having a pore diameter of 1 µm was used as the member B.

With respect to implanted samples implanted in rats, the penetration of a vital tissue into the member B and the degree of integration with a surrounding tissue were observed. As a result, the penetration of cells was good, and a capillary was also developed. In SEM observation, the vital tissue became completely continuous within the surrounding tissue and the porous wall, and no boundary line between a tissue within the wall and an outer tissue could be distinguished. The results are shown in Table 1.

### Example 4

An intracorporeally implanting material was produced in the same manner as in Example 1 except that a porous expanded PTFE sheet (product of Sumitomo Electric Industries, Ltd., trade name "Poreflon WP-045-80", thickness: about 80 µm, particle diameter capable of being captured: at least 0.45 µm) having a pore diameter of 0.45 µm was used as the member B.

With respect to implanted samples implanted in rats, the penetration of a vital tissue into the member B and the degree of integration with a surrounding tissue were observed. As a result, no penetration of cells was observed. In SEM observation, however, the penetration of thick fasciculi was confirmed. The results are shown in Table 1.

### Comparative Example 1

An intracorporeally implanting material was produced in the same manner as in Example 1 except that a porous expanded PTFE sheet (product of Sumitomo Electric Industries, Ltd., trade name "Poreflon WP-020-80", thickness: about 80 µm, particle diameter capable of being captured: at least 0.2 µm) having a pore diameter of 0.20 µm was used as the member B.

With respect to implanted samples implanted in rats, the penetration of a vital tissue into the member B and the degree of integration with a surrounding tissue were observed. As a result, no penetration of cells was observed. In SEM observation, however, the penetration of relatively thin fibers was confirmed. The results are shown in Table 1.

### Comparative Example 2

An intracorporeally implanting material was produced in the same manner as in Example 1 except that a porous expanded PTFE sheet (product of Sumitomo Electric Industries, Ltd., trade name "Poreflon WP-010-80", thickness: about 80 µm, particle diameter capable of being captured: at least 0.1 µm) having a pore diameter of 0.10 µm was used as the member B.

With respect to implanted samples implanted in rats, the penetration of a vital tissue into the member B and the degree of integration with a surrounding tissue were observed. As a result, no penetration of cells was observed. In SEM observation, however, the penetration of relatively thin fibers was confirmed. The results are shown in Table 1.

### Comparative Example 3

An intracorporeally implanting material was produced in the same manner as in Example 1 except that a porous expanded PTFE sheet (product of Sumitomo Electric Industries, Ltd., trade name "Poreflon WP-005-80", thickness: about 80 µm, particle diameter capable of being captured: at least 0.05 µm) having a pore diameter of 0.05 µm was used as the member B.

With respect to implanted samples implanted in rats, the penetration of a vital tissue into the member B and the degree of integration with a surrounding tissue were observed. As a result, no penetration of cells was observed. In SEM observation, however, the penetration of relatively thin fibers was confirmed. The results are shown in Table 1.

### Comparative Example 4

A commercially available patch composed of a vital tissue-blocking sheet (product of GORE Co., trade name "Goretex Pericardial EPTFE Patch II", thickness: 100 µm) was cut out into a form illustrated in Fig. 3(b). With respect to implanted samples implanted in rats, the penetration of a vital tissue into the member B and the degree of integration with a surrounding tissue were observed. As a result, no penetration of cells was observed. In SEM observation, however, the penetration of relatively thin fibers was confirmed. The results are shown in Table 1

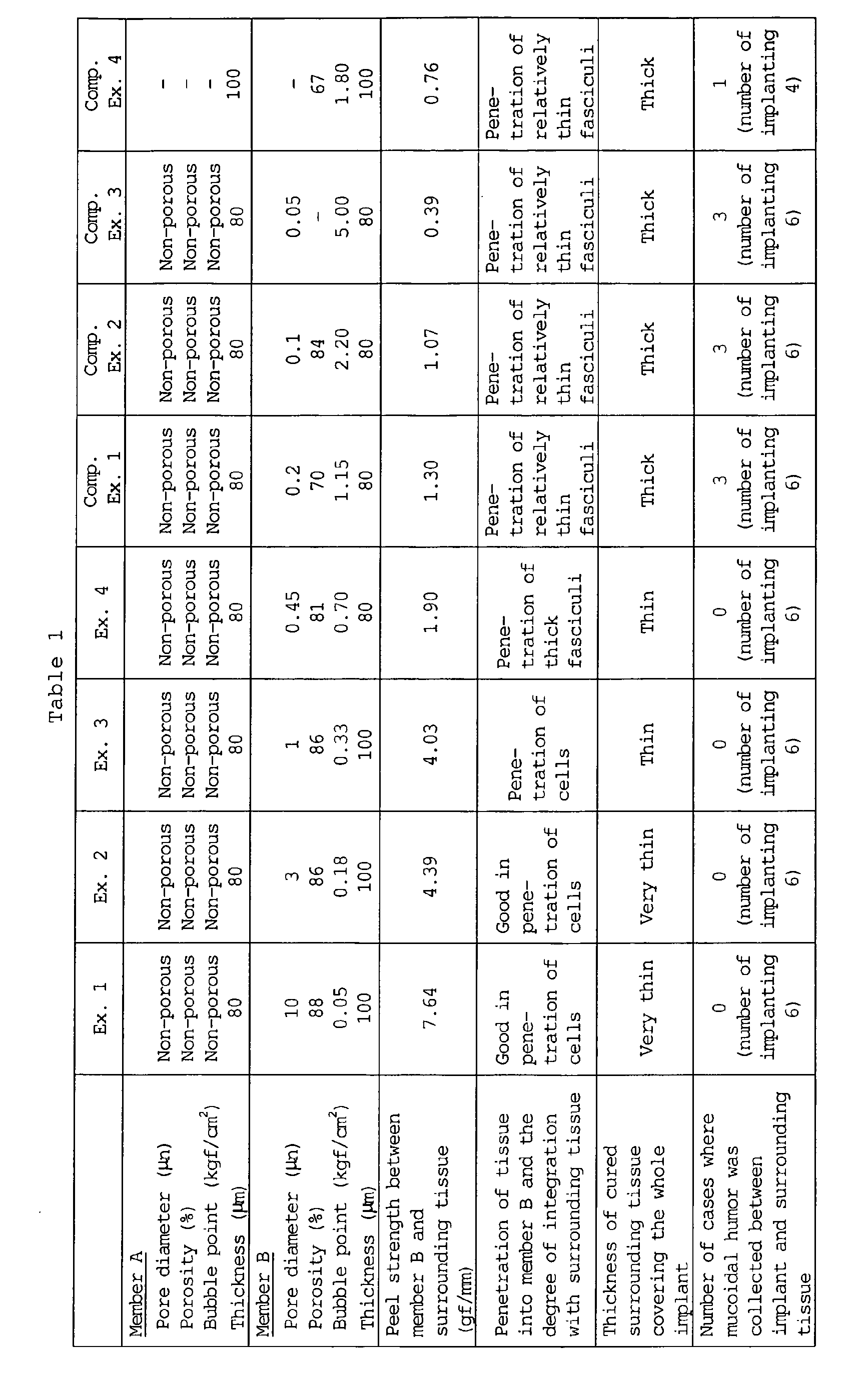

Although the results of the evaluation as to all the samples making use of the animals have been shown in Table 1, the cured tissues around the implants were thick in the samples (Comparative Examples 1 to 4) composed of only the member that is not intimate with the vital tissue and thus has the vital tissue-blocking ability, or obtained by combining such members into a composite body, and cases where the mucoidal humor was collected between the implant and the surrounding tissue amounted to about half the number.

On the other hand, in the samples (Examples 1 to 4) obtained by combining the member that is not intimate with the vital tissue and thus has the vital tissue-blocking ability, and the member that the vital tissue penetrates thereinto, and the surrounding tissue is integrated with and fixed to it, into a composite body, the surrounding cured tissue covering the whole implant was translucent and thin. Such samples exhibited anti-inflammatory property.

From the above-described fact, it is understood that a function that the cured tissue can be freely penetrated to be integrated with and fixed to the vital tissue is partially imparted to an intracorporeally implanting material having vital tissue-blocking ability, whereby inflammatory tendency can be greatly reduced.

### INDUSTRIAL APPLICABILITY

According to the present invention, there are provided intracorporeally implanting materials, which are each composed of a formed article of a synthetic resin, substantially have vital tissue-blocking ability and moreover can markedly inhibit inflammation caused by the hypertrophy of a cicatricial tissue and the retention of a humor at an implanted site and its surrounding site.

The intracorporeally implanting materials according to the present invention can achieve integration by partial penetration of a vital tissue while substantially retaining the vital tissue-blocking ability, thereby markedly inhibiting inflammation caused by the hypertrophy of a cicatricial tissue and the retention of a humor at an implanted site and its surrounding site. The respective formed resin articles are laminated into a composite body, whereby tear strength can be enhanced to make a suture operation easy. The formed porous resin article partially exposed can be integrated with the vital tissue, thereby firmly holding the intracorporeally implanting material within the vital body.

The intracorporeally implanting materials according to the present invention can be suitably utilized in uses of which the vital tissue-blocking ability is required, such as patch materials used for repairing a pericardium, pleura, diaphragm, peritoneum, tendovagina or the like.

## Claims

1. An intracorporeally implanting material comprising a composite body of a formed finely-porous or non-porous resin article (A), which is finely-porous, the pore diameter of which is smaller than 0.45 µm, or is non-porous and has vital tissue-blocking ability and a formed porous resin article (B), which is porous, the pore diameter of which is not smaller than 0.45 µm, and has vital tissue-penetrating ability, wherein the material has a structure that a part of the formed porous resin article (B) is exposed, and a vital tissue can be penetrated into the exposed part.

2. The intracorporeally implanting material according to claim 1, wherein the formed finely-porous or non-porous resin article (A) is a formed finely-porous resin article having a pore diameter smaller than 0.05 µm, or a formed non-porous resin article.

3. The intracorporeally implanting material according to claim 1, wherein the formed porous resin article (B) is a formed porous resin article having a pore diameter not smaller than 1 µm and vital tissue-penetrating ability.

4. The intracorporeally implanting material according to claim 1, wherein the formed porous resin article (B) is a formed porous resin article having a pore diameter not smaller than 3 µm and penetrability of a cell tissue and a capillary tissue penetrate.

5. The intracorporeally implanting material according to claim 1, wherein both formed finely-porous or non-porous resin article (A) and formed porous resin article (B) are formed resin articles formed from polytetrafluoroethylene.

6. The intracorporeally implanting material according to claim 5, wherein both formed finely-porous or non-porous resin article (A) and formed porous resin article (B) are porous expanded polytetrafluoroethylene bodies.

7. The intracorporeally implanting material according to claim 6, wherein the bubble point of the formed finely-porous or non-porous resin article (A) composed of the porous expanded polytetrafluoroethylene body is higher than 0.70 kgf/cm², and the bubble point of the formed porous resin article (B) composed of the porous expanded polytetrafluoroethylene body is not higher than 0.70 kgf/cm².

8. The intracorporeally implanting material according to claim 6, wherein the bubble point of the formed porous resin article (B) composed of the porous expanded polytetrafluoroethylene body is not higher than 0.50 kgf/cm².

9. The intracorporeally implanting material according to claim 6, wherein the bubble point of the formed porous resin article (B) composed of the porous expanded polytetrafluoroethylene body is not higher than 0.30 kgf/cm².

10. The intracorporeally implanting material according to claim 1, which is composed of a composite body obtained by laminating the formed finely-porous or non-porous resin article (A) and the formed porous resin article (B), which are both in the form of a sheet, and has a structure that a part of the sheet-like formed porous resin article (B) extends outside a peripheral edge portion of the sheet-like formed finely-porous or non-porous resin article (A) in an exposed state.

11. The intracorporeally implanting material according to claim 10, wherein the sheet-like formed finely-porous or non-porous resin article (A) is a sheet-like finely-porous expanded polytetrafluoroethylene body or formed non-porous polytetrafluoroethylene article.

12. The intracorporeally implanting material according to claim 10, wherein the sheet-like formed porous resin article (B) is a sheet-like porous expanded polytetrafluoroethylene body.

13. The intracorporeally implanting material according to claim 1, which is composed of a composite body obtained by laminating the formed finely-porous or non-porous resin article (A) and the formed porous resin article (B), which are both in the form of a sheet, and has a structure that a part of the sheet-like formed porous resin article (B) is exposed through an opening formed in the formed finely-porous or non-porous resin article (A).

14. The intracorporeally implanting material according to claim 13, wherein the sheet-like formed finely-porous or non-porous resin article (A) is a sheet-like finely-porous expanded polytetrafluoroethylene body or formed non-porous polytetrafluoroethylene article.

15. The intracorporeally implanting material according to claim 13, wherein the sheet-like formed porous resin article (B) is a sheet-like porous expanded polytetrafluoroethylene body.

16. The intracorporeally implanting material according to claim 1, which is composed of a composite body having a layer structure that a sheet-like formed porous resin article (B) is held between 2 sheet-like formed finely-porous or non-porous resin articles (A), and has a structure that a part of the sheet-like formed porous resin article (B) is exposed through openings respectively formed in the respective sheet-like formed finely-porous or non-porous resin articles (A).

17. The intracorporeally implanting material according to claim 16, wherein the sheet-like formed finely-porous or non-porous resin article (A) is a sheet-like finely-porous expanded polytetrafluoroethylene body or formed non-porous polytetrafluoroethylene article.

18. The intracorporeally implanting material according to claim 16, wherein the sheet-like formed porous resin article (B) is a sheet-like porous expanded polytetrafluoroethylene body.

19. The intracorporeally implanting material according to claim 1, wherein the color tone of the formed finely-porous or non-porous resin article (A) is made different from the color tone of the formed porous resin article (B) by coloring.
